# EUROPEAN PATENT APPLICATION

(11) **EP 0 819 677 A1**
(43) Date of publication of application: **21.01.1998**
(21) Application number: 97890091.8
(22) Date of filing: 21.05.1997
(51) Int. Cl.: C07D 215/18, C07D 471/06, C07C 309/19, C07B 57/00

(54) **Process for the preparation of optically active compounds**

(30) Priority: 21.05.1996 HU 9601361
(71) Applicant: CHINOIN Gyògyszer és Vegyészeti Termékek Gyára RT., H-1045 Budapest IV (HU)
(72) Inventor: Balint, Jozsef, Damjanich u. 2. (HU); Fetter, György, Landorhegi u. 24/A,I/5. (HU); Fogassy, Elemér, Ordögorom u. 20. (HU); Friesz, Antal, Buvar u. 1. (HU); Gajary, Antal, Bölöni Gy. u. 15/B. (HU)
(74) Representative: Schwarz, Albin, Dr. Kopecky & Schwarz Patentanwälte

(57) **Abstract**

Process for the resolution of 6-fluoro-1,2,3,4-tetrahydro-2-methyl-quinoline using L-(-)-α-bromocamphor-π-sulphonic acid as resolving agent.

## Description

This invention relates to the new optically active intermediate of formula (I) and its salts, the new salt of formula (VII), the optically pure compound of formula (II) and its salts, and the novel preparation process to obtain compounds of formulae (I) and (II) and their salts.

The racemic 6-fluoro-1,2,3,4-tetrahydro-2-methyl-quinoline and the racemic 9-fluoro-6,7-dihydro-5-methyl-1-oxo-1*H*,5*H*-pyrido[3,2,1-*i*,*j*]quinoline-2-carboxylic acid and its salts are known from the literature / German Patent Application publ. No. 2.264163 and French Patent Application publ. No. 2453647/. The latter pyridoquinoline derivative is used as an antibacterial agent in the human and veterinary medicine and in the course of cultivation of plants.

Our aim was to prepare the optically active compound of formula (II) and its salts -which possesses more favourable antibacterial properties than the above mentioned racemic pyridoquinoline - by an improved process and in higher optical purity.

We found that the resolution of the racemic 6-fluoro-1,2,3,4-tetrahydro-2-methyl-quinoline intermediate can be effected by applying L-(-)-α-bromocamphor-π-sulfonic acid salts, advantageously its ammonium salt. Resolution is carried out by treating the racemic 6-fluoro-1,2,3,4-tetrahydro-2-methyl-quinoline or its salt with the salt of the L-(-)-α-bromocamphor -π-sulphonic acid /[(1S)-(*endo,anti*)-(-)-3-bromocamphor-8-sulfonic acid / of formula (III), the resulting salt of formula (VII) is then isolated from the reaction mixture by a method known per se, if desired the salt is purified, occasionally the salt of formula (VII) is recovered from the purification mother liquor, and is purified, finally the (-)-6-fluoro-1,2,3,4-tetrahydro-2-methyl-quinoline of formula (I) is liberated from the salt of formula (VII), and if desired it can be transformed into an other salt.

Preparation of the salt of formula (VII) is performed in the presence of a solvent, preferably in aqueous solution, the resulting salt can then be purified by heating it in an organic solvent under reflux conditions. As for organic solvent for example ethyl acetate may be used. Liberation of the compound of formula (I) can be effected by use of a base, for example aqueous alkali metal hydroxide solution, and the compound of formula (I) may be isolated from the reaction mixture by extraction with an organic solvent, followed by evaporation. As for extracting solvent e.g. dichloromethane may be used.

The (+) and racemic 6-fluoro-1,2,3,4-tetrahydro-2-methyl-quinolines which remain dissolved in the mother liquor may be recovered by various ways.

One possibility is to transform the racemic 6-fluoro-1,2,3,4-tetrahydro-2-methyl-quinoline, which remained in the mother liquors, into its hydrochloride salt and remove the (+)-6-fluoro-1,2,3,4-tetrahydro-2-methyl-quinoline from the mixture by distillation in vacuo, occasionally the hydrochloride salt formation and the distillation may repeatedly be performed, both with the distilled material and with the distillation residue, one or more times.

According to another method the racemic 6-fluoro-1,2,3,4-tetrahydro-2-methyl-quinoline and the (+)-6-fluoro-1,2,3,4-tetrahydro-2-methyl-quinoline remaining dissolved in the mother liquor are separated from each other by creating conditions where their partitions between the solid and the solution phases are different.

According to this method either the (+)-6-fluoro-1,2,3,4-tetrahydro-2-methyl-quinoline is taken into the solid phase, by lowering the temperature, or the racemic 6-fluoro-1,2,3,4-tetrahydro-2-methyl-quinoline is precipitated, by adding aqueous hydrochloric acid to the mixture. Carrying out the above procedures, if needed repeatedly, the optically pure (+)-6-fluoro-1,2,3,4-tetrahydro-2-methyl-quinoline and the unchanged racemic compound may be obtained separately. The racemic compound may then be resolved, whereas the (+) enantiomer may be racemized. The above two methods significantly improve the effectiveness of the present process and owing to them the use of further foreign chiral reagents can be avoided. The optically active compound of general formula (I) is then transformed into the optically pure compound of general formula (II) by applying the method described below.

The (-)-6-fluoro-1,2,3,4-tetrahydro-2-methyl-quinoline of formula (I) is reacted with the dialkyl (alkoxymethylene)malonate of general formula (IV) -wherein R is a C₁₋₄ alkyl group - and the resulting diester of general formula (V) -wherein R is a C₁₋₄ alkyl group - is cyclized into the carboxylic acid ester of general formula (VI) - wherein R has the same meaning as above - which is then transformed by hydrolysis into the compound of formula (II) and, if desired a salt of the compound of formula (II) may be formed.

From the compounds of general formula (IV) the use of diethyl (ethoxymethylene)malonate is advantageous. The ethanol which is formed in the condensation reaction is distilled off during the reaction, the temperature of the reaction is favourably 110-130°C.

Ring closure of the compounds of general formula (V) may preferably be performed in the presence of polyphosphoric acid, which is added to the cooled (20°C) reaction mixture, the temperature is then raised to 100-120°C. Hydrolysis of the esters of general formula (VI) is effected at reflux temperature and the resulting compound of formula (II), which precipitates from the reaction mixture after cooling may be separated from the mixture by a method known per se. The raw product of formula (II) may be purified by recrystallisation, preferably e.g. from dimethyl formamide.

In the course of the preparation of the compound of formula (II) the intermediates of general formulae (V) and (VI) are preferably not isolated.

During the above multi-step synthesis, surprisingly, the optical purity does not deteriorate, racemization and degradation do not take place, thus the targeted compound of formula (II) can be prepared in an enantiomeric excess of 98%, at the least. The optical purity plays important role in the biological behaviour of optically active compounds /Chirality **5**, p. 350-355(1993)/.
Further details of our invention are shown by the examples below, without limiting our the claims to the examples.

### Examples

### Example 1

a.) 3,75 g (22,70 mmol) of racemic 6-fluoro-1,2,3,4-tetrahydro-2-methyl-quinoline, 1,90 ml of 37% hydrochloric acid (d = 1,18 g/cm³) (22,74 mmol) , 5,00 g (15,23 mmol) of L-(-)-α-bromocamphor-π-sulfonic acid ammonium salt and 30 ml of water were mixed. The mixture was heated till a solution was obtained. On cooling an oily phase precipitated which slowly crystallized on scraping. The mixture was allowed to stay at 10-15°C for 1 hour, then the crystals were collected and washed with 3 x 2 ml of water. The mother liquor (A0) was saved.
b.) The wet collected crystalline material (9.0 g) was heated in 10 ml of ethyl acetate under reflux conditions for 10 mins, then cooled down to -10°C and kept at that temperature for 1 hour. The precipitated crystalline material was collected and washed with 3 x 1 ml of cold ethyl acetate. The mother liquor (A1) was saved. The crystalline material, the sulfonic acidic salt, was dried until constant mass. Thus 2.78 g (5.84 mmol) of (-)-6-fluoro-1,2,3,4-tetrahydro-2-methyl-quinoline L-(-)-α-bromocamphor-π-sulfonic acid salt was obtained.
   The above salt was taken up in 10 ml of water, the suspension was made alkaline by adding 2.0 ml of 8 mol/l concentration sodium hydroxide solution. The aqueous phase was extracted with 3 x 10 ml of dichloromethane. The combined organic layers were dried over sodium sulfate, filtered and evaporated in vacuo. Thus in a yield of 46.4%, 0.87 g (5.27 mmol) of (-)-6-fluoro-1,2,3,4-tetrahydro-2-methyl-quinoline of formula (I) were obtained in the form of an oil, which crystallized following inoculation. [α]_{D}^{RT}= -60.4° (c = 1, ethanol).
c.) The mother liquor (A0) of the resolution was made alkaline with 2.0 ml of 8 mol/ l contrentration sodium hydroxide solution, the aqueous phase was extracted with 3 x 10 ml of dichloromethane. The combined organic phases were dried over sodium sulfate, filtered and evaporated in vacuo. Thus, 1.64 g (9.93 mmol, 87.5%,) oily material was obtained, which following inoculation stiffened into oily crystals, consisting of (+)-6-fluoro-1,2,3,4-tetrahydro-2-methyl-quinoline and racemic 6-fluoro-1,2,3,4-tetrahydro-2-methyl-quinoline [α]_{D}^{RT} = +24.2° (c = 1, ethanol).
d.) From the mother liquor (A1) of the purification ethyl acetate was removed in vacuo. To the residual 4.0 g of solid material 15 ml of water was added and the mixture was made alkaline by addition of 8 mol/ l concentration sodium hydroxide solution. The aqueous phase was extracted with 3 x 10 ml of dichloromethane. The combined organic phases were dried on sodium sulfate, filtered and evaporated in vacuo. Thus, 1.21 g (7.32 mmol, 64.5%,) oil was obtained, which following inoculation stiffened into oily crystals, consisting of the mixture of the racemic and the (+) 6-fluoro-1,2,3,4-tetrahydro-2-methyl-quinoline [α]_{D}^{RT} = +13.5 ° (c = 1, ethanol).

### Example 2.

1.00 g of (-)-6-fluoro-1,2,3,4-tetrahydro-2-methyl-quinoline [α]_{D}^{RT} = -70.0° (enantiomeric excess >98%, c = 1, ethanol) was heated with 1.56 g of diethyl (ethoxymethylene)malonate. After the ethanol distillation has ceased, the reaction apparatus was kept under vacuo for 5 minutes, using an ejector-jet pump. The hot, 120°C reaction mixture was then cooled to room temperature, 1.50 g of polyphosphoric acid was added, and the mixture was stirred and heated at 110°C for 5 hours and 30 mins. Following the addition of 3 ml of water it was further heated at 110 °C under reflux conditions for another 2 hours. The mixture was cooled down to room temperature and the crystals of the resulting thick suspension were collected, washed subsequently with 5 x 1 ml of water and 3 x 0.25 ml of ethanol. The wet raw product was recrystallized from 2 ml of dimethyl formamide, the crystals were collected, washed subsequently with 2 x 0.25 ml of dimethyl formamide, 3 x 1 ml of water and 2 x 0.25 ml of ethanol, then they were dried to constant mass. Thus 1.2 g (75.9 %) white crystals of (-)-9-fluoro-6,7-dihydro-5-methyl-1-oxo-1*H*,5*H*-pyrido[3,2,1-*i*,*j*]quinoline-2-carboxylic acid /(-)-flumequin/ was obtained, enantiomeric excess: >98%, [α]_{D}^{RT} = -48.9° (c = 0.24, dimethyl formamide), mp.: 245-248°C.

### Example 3.

34.6 g (209.4 mmol) of 6-fluoro-1,2,3,4-tetrahydro-2-methyl-quinoline [α]_{D}²⁶ = +31.75° (c = 1, ethanol) was dissolved in 35 ml of ethanol. Under cooling 9.6 ml (114.9 mmol) of 37% hydrochloric acid (d = 1.18) was added. The solvent was removed in vacuo, the residue was distilled. The fraction obtained around 130°C/1.6 kPa was rich in the (+) enantiomer. This fraction, 14.22 g (86.1 mmol, 41.1%) [α]_{D}²⁶ = +41.9° (c = 1, ethanol), was an oil which stiffened into oily crystals following inoculation.
To the solid residue of the distillation 70 ml of water and 5.0 g (125 mmol) of sodium hydroxide was added and the solution was extracted with 3 x20 ml of dichloromethane. The combined organic phases were dried over sodium sulfate, filtered and evaporated. The residue was an oil, 19.04 g (115.2 mmol, 55.0%), [α]_{D}²⁶ = +22.4° (c = 1, ethanol). The oil stiffened into oily crystals following inoculation.

### Example 4.

To 66.0 g (399.5 mmol) of 6-fluoro-1,2,3,4-tetrahydro-2-methyl-quinoline [α]_{D}²⁰ = +7.2° (c = 1, ethanol) 200 ml of water and 35 ml of 37% hydrochloric acid (419.0 mmol, d = 1.18) were added and the mixture was heated until dissolution. From the clear solution the crystallization started on cooling and scraping. The resulting suspension was cooled down to 20°C, the crystals were collected, washed with 3 x 15 ml of water. The thus obtained wet crystals of 6-fluoro-1,2,3,4-tetrahydro-2-methyl-quinoline hydrochloride were taken up in 150 ml of water. To the suspension 13.0 g (325.0 mmol) of sodium hydroxide was added and the mixture was extracted with 3 x 50 ml of dichloromethane. The organic phases were combined, dried over sodium sulfate and filtered. Dichloromethane was removed in vacuo to obtain 48.0 g (290.5 mmol, 72.7 %) of 6-fluoro-1,2,3,4-tetrahydro-2-methyl-quinoline base [α]_{D}²⁰ = +1.0° (c = 1, ethanol).

To the mother liquor of the hydrochloride salt 7.0 g (175.0 mmol) of sodium hydroxide was added and the mixture was extracted with 3 x 50 ml of dichloromethane. The organic layers were combined, dried over sodium sulfate. Dichloromethane was removed in vacuo to obtain 13.9 g (84.1 mmol, 21.1%) of 6-fluoro-1,2,3,4-tetrahydro-2-methyl-quinoline base [α]_{D}²⁰ = +25.8° (c = 1, ethanol).

### Example 5.

By a method similar as described in Example 4, starting from 130.0 g (786.7 mmol) of 6-fluoro-1,2,3,4-tetrahydro-2-methyl-quinoline with optical rotation [α]_{D}²⁰ = +29.7° (c = 1, ethanol), by preparing and collecting the crystals of the 6-fluoro-1,2,3,4-tetrahydro-2-methyl-quinoline hydrochloride, and liberating the base, 66.5 g (402.5 mmol, 51.2 %) of 6-fluoro-1,2,3,4-tetrahydro-2-methyl-quinoline base [α]_{D}²⁰ = +7.2° (c = 1, ethanol) was obtained, whereas by working up the mother liquor of the hydrochloride salt, 57.2 g (346.2 mmol, 44.0 %) of [α]_{D}²⁰ = +57.4° (c = 1, ethanol) 6-fluoro-1,2,3,4-tetrahydro-2-methyl-quinoline base was obtained.

### Example 6.

11.5 g (69.6 mmol) of 6-fluoro-1,2,3,4-tetrahydro-2-methyl-quinoline base [α]_{D}²⁰ = + 35.4° (c = 1, ethanol) was cooled to 0 - 5°C, and the oil was seeded with the crystals of the (+)-6-fluoro-1,2,3,4-tetrahydro-2-methyl-quinoline enantiomer. The resulting crystalline mass was crushed, it was placed on a glass filter previously cooled to approximately 0°C, and it was filtered, while the temperature was allowed to gradually raise to ambient (approx. 22°C). The material collected on the filter, 1.2 g (7.3 mmol, 10.4%), was the crystalline 6-fluoro-1,2,3,4-tetrahydro-2-methyl-quinoline base [α]_{D}²⁰ = +64.8° (c = 1, ethanol), whereas the filtrate, 9.9 g (59.9 mmol, 86.1%) consisted of the oily 6-fluoro-1,2,3,4-tetrahydro-2-methyl-quinoline base [α]_{D}²⁰ = +32.8° (c = 1, ethanol).

### Example 7.

By a method similar as described in Example 6, starting from 14.5 g (87.8 mmol) of 6-fluoro-1,2,3,4-tetrahydro-2-methyl-quinoline base [α]_{D}²⁰ = +40.3° (c = 1, ethanol), the material collected on the filter, 2.4 g (14.5 mmol, 16.6 %) was the white, crystalline 6-fluoro-1,2,3,4-tetrahydro-2-methyl-quinoline base, [α]_{D}²⁰ = +65.2° (c = 1, ethanol). The filtrate, 12.0 g (72.6 mmol, 82.8%) consisted of the oily [α]_{D}²⁰ = +35.4° (c = 1, ethanol) 6-fluoro-1,2,3,4-tetrahydro-2-methyl-quinoline base.

### Example 8.

By a method similar as described in Example 6, starting from 22.0 g (133.2 mmol) of 6-fluoro-1,2,3,4-tetrahydro-2-methyl-quinoline base [α]_{D}²⁰ = + 48.8°.(c = 1, ethanol), the material collected on the filter, 6.5 g (39.3 mmol, 29.5%) was the white, crystalline 6-fluoro-1,2,3,4-tetrahydro-2-methyl-quinoline base [α]_{D}²⁰ = +65.8° (c = 1, ethanol). The filtrate, 15.1 g (91.4 mmol, 68.6%) consisted of the oily [α]_{D}²⁰ = +40.3° (c = 1, ethanol) 6-fluoro-1,2,3,4-tetrahydro-2-methyl-quinoline base.

### Example 9.

55.0 g (332.9 mmol) of oily [α]_{D}²⁰ = +57.4° (c = 1, ethanol) 6-fluoro-1,2,3,4-tetrahydro-2-methyl-quinoline base was seeded at room temperature (approx. 22°C ) with the crystals of the (+)-6-fluoro-1,2,3,4-tetrahydro-2-methyl-quinoline enantiomer. (In case the crystallization did not start, the mixture was cooled to 10 - 15°C.) The mixture was allowed to crystallize at room temperature for 1 hour, then it was filtered. The material collected on the filter, 31.9 g (193.1 mmol, 58.0%), was the crystalline 6-fluoro-1,2,3,4-tetrahydro-2-methyl-quinoline base [α]_{D}²⁰ = +65.4° (c = 1, ethanol The filtrate, 22.9 g (138.6 mmol, 41.6%) consisted of the oily 6-fluoro-1,2,3,4-tetrahydro-2-methyl-quinoline base [α]_{D}²⁰ = +48.8° (c = 1, ethanol).

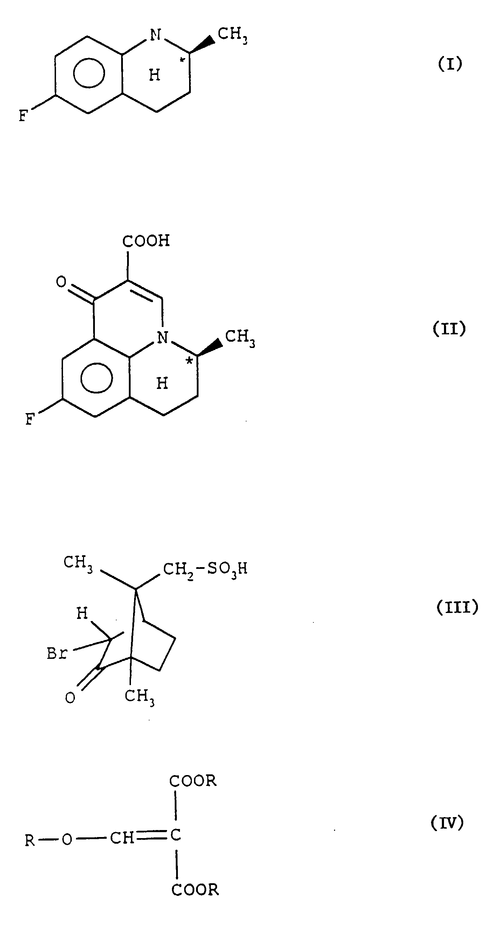

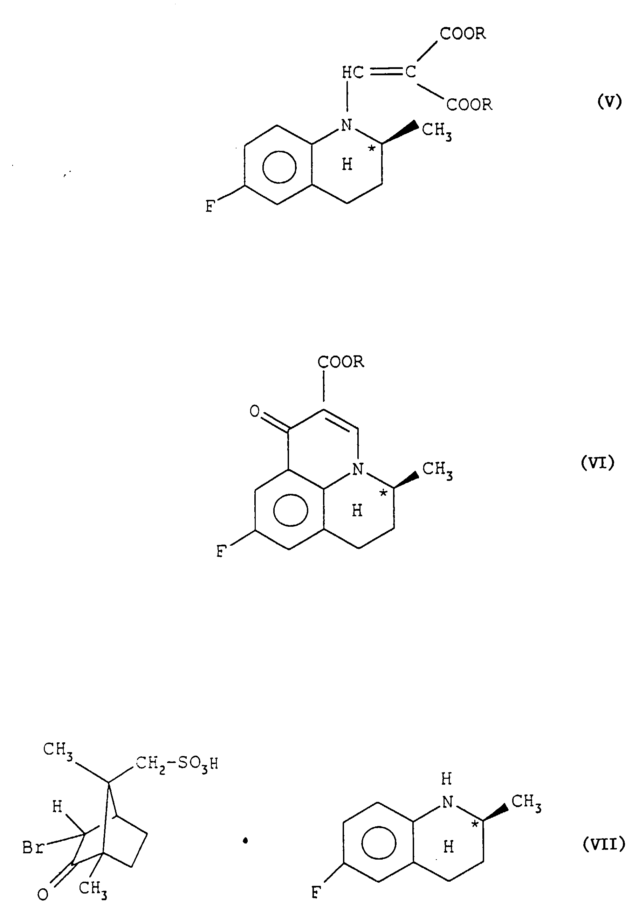

## Claims

1. Process for the preparation of the (-)-6-fluoro-1,2,3,4-tetrahydro-2-methyl-quinoline of general formula (I) and its salts **characterized by** that the racemic 6-fluoro-1,2,3,4-tetrahydro-2-methyl-quinoline or its salt is resolved with the L-(-)-α-bromocamphor-π-sulfonic acid of general formula (III) or its salt.

2. Process, according to claim 1., for the preparation of the (-)-6-fluoro-1,2,3,4-tetrahydro-2-methyl-quinoline of general formula (I) and its salts **characterized by** that the racemic 6-fluoro-1,2,3,4-tetrahydro-2-methyl-quinoline or its salt is reacted with the L-(-)-α-bromocamphor-π-sulfonic acid salt of general formula (III), the salt of formula (VII) is isolated from the reaction mixture by a method known per se, if desired it is purified, occasionally the salt of formula VII is isolated also from the purification mother liquor, and is purified, and from the salt of formula (VII) the (-)-6-fluoro-1,2,3,4-tetrahydro-2-methyl-quinoline of formula (I) is liberated, and if desired transformed into an other salt.

3. Process, according to claim 1., **characterized by** that the compound of general formula (III) is applied in the form of its ammonium salt.

4. Process, according to claim 1., **characterized by** that the racemic 6-fluoro-1,2,3,4-tetrahydro-2-methyl-quinoline is applied in the form of its hydrochloride salt.

5. Process, according to claim 1., **characterized by** that the formation of the camphorsulfonic acid salt of formula (VII) is carried out in aqueous medium.

6. Process, according to claim 1., **characterized by** that the racemic 6-fluoro-1,2,3,4-tetrahydro-2-methyl-quinoline is transformed in aqueous medium into its hydrochloride salt, reacted with L-(-)-α-bromocamphor-π-sulfonic acid ammonium salt, the precipitated (-)-6-fluoro-1,2,3,4-tetrahydro-2-methyl-quinoline camphorsulfonic acid salt of general formula (VII) is isolated, purified in refluxing ethyl acetate, then the compound of formula (I) is liberated from the salt on the effect of an aqueous base and it is isolated from the reaction mixture.

7. Process, according to claim 1., **characterized by** that the racemic 6-fluoro-1,2,3,4-tetrahydro-2-methyl-quinoline, remaining in the mother liquors, is transformed into its hydrochloride salt and the (+)-6-fluoro-1,2,3,4-tetrahydro-2-methyl-quinoline is separated from it by distilling it off in vacuo, and occasionally the hydrochloride salt formation and the distillation may be carried out repeatedly, from both the distilled material and the distillation residue, one or more times.

8. Process, according to claim 1., **characterized by** that the racemic 6-fluoro-1,2,3,4-tetrahydro-2-methyl-quinoline and the (+)-6-fluoro-1,2,3,4-tetrahydro-2-methyl-quinoline, remaining in the mother liquors, are separated from their mixture by creating conditions where their partitions between the solid and the solution phases are different.

9. Process, according to claim 8., **characterized by** that by lowering the temperature the (+)-6-fluoro-1,2,3,4-tetrahydro-2-methyl-quinoline is taken into the solid phase.

10. Process, according to claim 8., **characterized by** that by the addition of aqueous hydrochloric acid the racemic 6-fluoro-1,2,3,4-tetrahydro-2-methyl-quinoline is taken into the solid phase, in the form of its hydrochloride salt.

11. Process for the preparation of the (-)-9-fluoro-6,7-dihydro-5-methyl-1-oxo-1*H*,5*H*-pyrido[3,2,1-*i*, *j*]quinoline-2-carboxylic acid of formula (II), **characteriz ed by** that the (-)-6-fluoro-1,2,3,4-tetrahydro-2-methyl-quinoline of formula (I) is reacted with the dialkyl (alkoxymethylene)malonate of general formula (IV) -wherein R is a C₁₋₄ alkyl group - and the resulting diester of general formula (V) -wherein R is a C₁₋₄ alkyl group - is cyclized into the carboxylic ester of general formula (VI) - wherein R has the same meaning as given above - which is then transformed by hydrolysis into the compound of formula (II) and, if desired a salt of the compound of formula (II) is formed.

12. Process, according to claim 11., **characterized by** that the cyclization is carried out in the presence of polyphosphoric acid.

13. The (-)-6-fluoro-1,2,3,4-tetrahydro-2-methyl-quinoline of formula (I) and its salts.

14. The (-)-6-fluoro-1,2,3,4-tetrahydro-2-methyl-quinoline of formula (I) in its essentially optically pure form.

15. The (-)-6-fluoro-1,2,3,4-tetrahydro-2-methyl-quinoline L-(-)-α-bromocamphor-π-sulfonic acid salt of formula (VII).

16. The (-)-9-fluoro-6,7-dihydro-5-methyl-1-oxo-1*H*,5*H*-pyrido[3,2,1-*i*,*j*] quinoline-2-carboxylic acid of formula (II) in its essentially optically pure form.
